# EUROPÄISCHE PATENTSCHRIFT

(11) **EP 1 005 855 B1**
(45) Veröffentlichungstag und Bekanntmachung des Hinweises auf die Patenterteilung: **04.06.2003**
(21) Anmeldenummer: 99122884.2
(22) Anmeldetag: 18.11.1999
(51) Int. Cl.: A61K 7/42, A61K 31/275, C07C 255/37

(54) **Dimere Alpha-Alkyl-Styrolderivate als photostabile UV-Filter in kosmetischen und pharmazeutischen Zubereitungen**
Dimer alpha-alkyl-styrene derivatives as photostable UV-filters for cosmetic or pharmaceutical compositions
Dérivés dimères de alpha-alkyl-styrène comme filtres UV photostables pour préparations cosmétiques et pharmaceutiques

(30) Priorität: 03.12.1998 DE 19855649
(43) Veröffentlichungstag der Anmeldung: 07.06.2000
(73) Patentinhaber: BASF AKTIENGESELLSCHAFT, 67056 Ludwigshafen (DE)
(72) Erfinder: Habeck, Thorsten, Dr., 67149 Meckenheim (DE); Prechtl, Frank, Dr., 60318 Frankfurt (DE); Wünsch, Thomas, Dr., 67346 Speyer (DE); Westenfelder, Horst, 67435 Neustadt (DE)

(56) Entgegenhaltungen:
- EP-A- 0 251 398
- EP-A- 0 273 280
- EP-A- 0 401 128
- EP-A- 0 413 648
- FR-A- 2 658 075
- GB-A- 1 115 596
- US-A- 3 275 520
- US-A- 4 387 089

## Beschreibung

Die Erfindung betrifft die Verwendung von substituierten, dimeren α-Alkyl-Styrolderivaten als photostabile UV-Filter in kosmetischen und pharmazeutischen Zubereitungen zum Schutz der menschlichen Epidermis oder menschlicher Haare gegen Sonnenstrahlen, speziell gegen UV-Strahlung im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-Aund UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

EP-A-0 413 648 beschreibt die Verwendung von dimeren, substituierten Styrolderivaten als UV-Absorber in kosmetischen Sonnenschutzmitteln.

EP-A-0 401 128, DE-A-1 568 693 und US 4,749,774 beschreiben jeweils die Verwendung von dimeren, substituierten Styrolderivaten als UV-Absorber in Kunststoffen beispielsweise in Verpackungsmaterialien für Getränke und Lebensmitteln.

Die in den oben genannten Patentschriften offenbarten, dimeren Styrolderivate weisen jedoch eine nicht zufriedenstellende Photostabilität auf.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und je nach Substituent in Öl oder Wasser löslich sind.

Diese Aufgabe wurde erfindungsgemäß gelöst durch Verwendung von substituierten, dimeren α-Alkyl-Styrolderivaten der Formel I 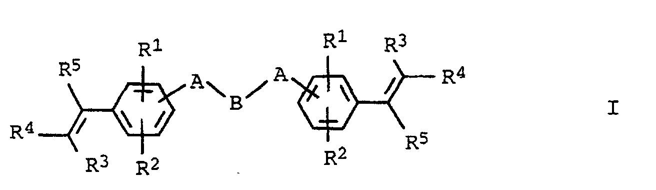 in der die Substituenten unabhängig voneinander folgende Bedeutung haben:
- R¹ und R²: Wasserstoff, OH, NH₂, C₁-C₁₂-Alkyl, C₁-C₁₂-Alkoxy, C₁-C₁₂-Alkylamino, C₁-C₁₂-Dialkylamino, wasserlöslich machende Substituenten, ausgewählt aus der Gruppe bestehend aus Carboxylat-, Sulfonat- oder Ammoniumresten;
- R³: COOR⁶, COR⁶, CONR⁶R⁷, CN;
- R⁴: COOR⁶, COR⁶, CONR⁶R⁷, CN;
- R⁵: C₁-C₁₂-Alkyl,
- R⁶ und R⁷: Wasserstoff, C₁-C₁₂-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
- A: O, S, N-R⁸;
- B: C₁-C₁₂-Alkylen, C₄-C₁₂-Cycloalkylen, C₈-C₂₂-Aralkylen, C₉-C₂₁-Monooxoaralkylen, [-X-]ₙ-Y-;
- R⁸: Wasserstoff, C₁-C₁₂-Alkyl;
- X: -CH₂-CH₂-Z-, -CH₂-CH₂-CH₂-Z-, -CH(CH₃)-CH₂-Z-, -CH₂-CH₂-CH₂-CH₂-Z-, -CH₂-CH₂-CH₂-CH₂-Z-,
- Y: -CH₂-CH₂-, -CH₂-CH₂-CH₂-, -CH(CH₃)-CH₂-, -CH₂-CH₂-CH₂-CH₂-, - CH₂-CH(CH₂-CH₃)-;
- Z: O, S;
- n: 1 bis 150,
wobei jeweils zwei der Reste R¹, R² und R⁸ zusammen mit dem aromatischen Ring, an dem sie gebunden sind, einen 5- oder 6-Ring bilden können, als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Alkylreste für R¹ und R² sowie für R⁶ bis R⁸ seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1, 2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl und n-Dodecyl genannt.

Als Alkenylreste für R⁶ und R⁷ seien verzweigte oder unverzweigte C₂-C₁₀-Alkenylketten, bevorzugt Ethenyl, n-Propenyl, 1-Methylethenyl, n-Butenyl, 1-Methylpropenyl-, 2-Methylpropenyl, 1,1-Dimethylethenyl, n-Pentenyl, 1-Methylbutenyl, 2-Methylbutenyl, 3-Methylbutenyl, 2,2-Dimethylpropenyl, 1-Ethylpropenyl, n-Hexenyl, 1.1-Dimethylpropenyl, 1,2-Dimethylpropenyl, 1-Methylpentenyl, 2-Methylpentenyl, 3-Methylpentenyl, 4-Methylpentenyl, 1,1-Dimethylbutenyl, 1,2-Dimethylbutenyl, 1,3-Dimethylbutenenyl, 2,2-Dimethylbutenyl, 2,3-Dimethylbutenyl, 3,3-Dimethylbutenyl, 1-Ethylbutenyl, 2-Ethylbutenyl, 1,1,2-Trimethylpropenyl, 1,2,2-Trimethylpropenyl, 1-Ethyl-1-methylpropenyl, 1-Ethyl-2-methylpropenyl, n-Heptenyl, n-Octenyl, n-Nonenyl, n-Decenyl genannt.

Als Cycloalkylreste seien für R⁶ und R⁷ verzweigte oder unverzweigte C₃-C₁₀-Cycloalkylketten, bevorzugt Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1 -Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Als Cycloalkenylreste seien für R⁶ und R⁷ verzweigte oder unverzweigte C₃-C₁₀-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecyl genannt.

Die Cycloalkenyl- und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R¹ und R² kommen solche mit 1 bis 12 C-Atomen, vorzugsweise mit 4 bis 8 C-Atomen in Betracht.

Beispielsweise sind zu nennen:

| | |
|---|---|
| Methoxy- | Ethoxy- |
| n-Propoxy- | iso-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Mono- oder Dialkylaminoreste für R¹ und R² kommen solche in Betracht, die Alkylreste mit 1 bis 12 C-Atomen, bevorzugt 1 bis 8 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl für R⁶ und R⁷ sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Heteroaryl-Reste für R⁶ und R⁷ sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Hydrophile d.h. die Wasserlöslichkeit der Verbindungen der Formel I ermöglichende Reste für R¹ und R² sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Ammoniumreste, insbesondere Alkylammoniumreste mit beliebigen physiologisch verträglichen Anionen in Betracht.

Als Alkylenreste für B seien verzweigte oder unverzweigte C₁-C₁₂-Alkylenketten, bevorzugt Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1, 1-Dimethylpropylen, 1,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen, n-Nonylen, n-Decylen, n-Undecylen und n-Dodecylen genannt.

Die o.g. Alkylenreste können gegebenenfalls einfach oder mehrfach substituiert sein. Als mögliche Substituenten seien dabei folgende Gruppen genannt: OH, O-C₁-C₆-Acyl, O-C₁-C₆-Alkyl, NH₂, NH-C₁-C₆-Alkyl, NH-C₁-C₆-Acyl, CN, COOH, COO-C₁-C₆-Alkyl.

Als Cycloalkylenreste für B seien C₄-C₁₂-Cycloalkylenreste, beispielsweise 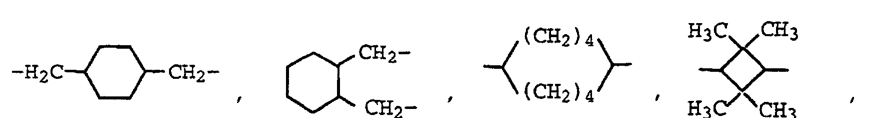 genannt.

Als Aralkylenreste für B seien C₈-C₂₂-Aralkylenreste, beispielsweise 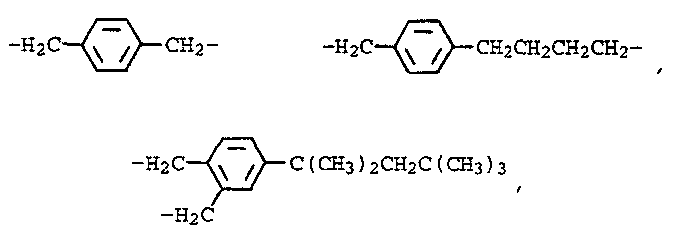 genannt.

Als Monooxoaralkylenreste für B seien C₉-C₂₁-Monooxoaralkylenreste, beispielsweise 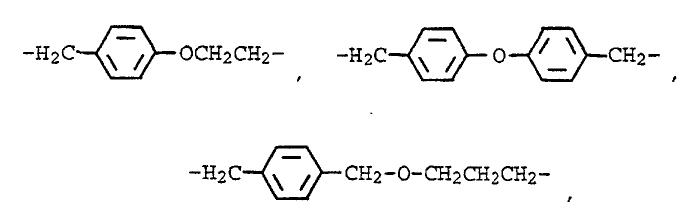 genannt .

Der Rest [-X-]ₙ-Y- steht für dimere oder polymere C₂-C₄-Alkylether, C₂-C₄-Alkylthioether oder C₂-C₄-Alkylimine mit 2 bis 151 alternierenden Monomereinheiten. Bevorzugt sind dimere oder polymere C₂-C₄-Alkylether mit 2 bis 21 alternierenden Monomereinheiten, ganz besonders bevorzugt dimere oder polymere Ethylenglykole oder Propylenglykole mit 2 bis 21 alternierenden Monomereinheiten.

Jeweils zwei der Reste R¹, R² und R⁸ können zusammen mit dem aromatischen Ring, an dem sie gebunden sind, einen weiteren 5- oder 6-Ring bilden. Als Beispiel seien folgende Strukturen genannt:

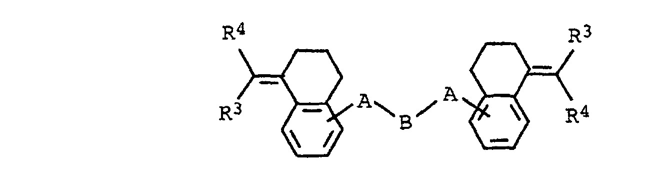

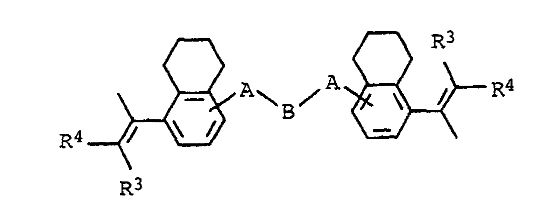

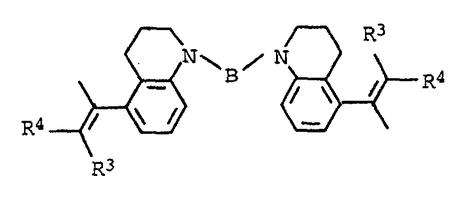

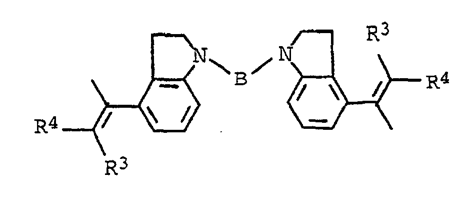

Bevorzugt sind solche Verbindungen der Formel I, in der
- R¹ und R²: Wasserstoff, C₁-C₈-Alkyl, C₁-C₈-Alkoxy, wasserlöslich machende Substituenten, ausgewählt aus der Gruppe bestehend aus Carboxylat-, Sulfonat- oder Ammoniumresten;
- R³: COOR⁶, CN;
- R⁴: COOR⁶, CN;
- R⁵: C₁-C₆-Alkyl;
- R⁶: C₁-C₁₂-Alkyl;
- A: O;
- B: C₁-C₁₂-Alkylen, C₈-C₂₂-Aralkylen, C₄-C₁₂-Cycloalkylen, [-X-]ₙ-Y;
- X: -CH₂-CH₂-O-, -CH₂-CH₂-CH₂-O-, -CH(CH₃)-CH₂-O-, -CH₂-CH₂-CH₂-CH₂-O-, -CH₂-CH(CH₂-CH₃)-O- ;
- Y: -CH₂-CH₂-, -CH₂-CH₂-CH₂-, -CH(CH₃)-CH₂-, -CH₂-CH₂-CH₂-CH₂-, -CH₂-CH(CH₂-CH₃)-;
- n: 1 bis 20
bedeutet.

Besonders bevorzugt sind solche Verbindungen der Formel I, in der
- R¹ und R²: Wasserstoff, C₄-C₈-Alkyl, C₄-C₈-Alkoxy, wasserlöslich machende Substituenten, ausgewählt aus der Gruppe bestehend aus Carboxylat-, Sulfonat- oder Ammoniumresten;
- R³ und R⁴: CN;
- R⁵: C₁-C₆-Alkyl;
- A: O;
- B: C₁-C₁₂-Alkylen, [-X-]ₙ-Y;
- X: -CH₂-CH₂-O-, -CH (CH₃ ) -CH₂-O-;
- Y: -CH₂-CH₂-, -CH(CH₃)-CH₂-;
- n: 1 bis 20
bedeutet.

Die Gruppe -A-B-A- kann jeweils in ortho, meta oder para Position am Aromaten gebunden sein, bezogen auf den, R³ bis R⁵ enthaltenden Rest. Besonders bevorzugt sind Verbindungen der Formel I, in denen -A-B-A- in der para-Position vorliegt.

Ganz besonders bevorzugt sind solche substituierten, dimeren α-Alkyl-Styrolderivaten mit der folgenden Strukturformel Ia, 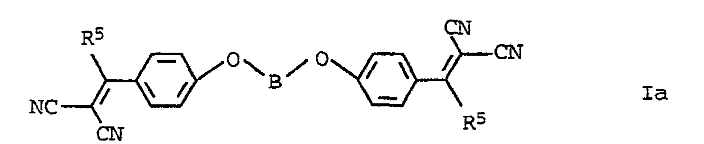 wobei die Substituenten folgende Bedeutung haben:
- R⁵: C₁-C₆-Alkyl, bevorzugt Methyl;
- B: C₁-C₁₂-Alkylen.

So weisen die folgenden, in der Tabelle 1 aufgeführten Verbindungen der Formel Ia besondere photostabile Eigenschaften aus. Tabelle 1:

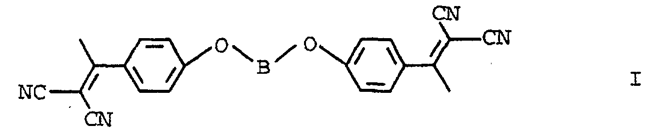

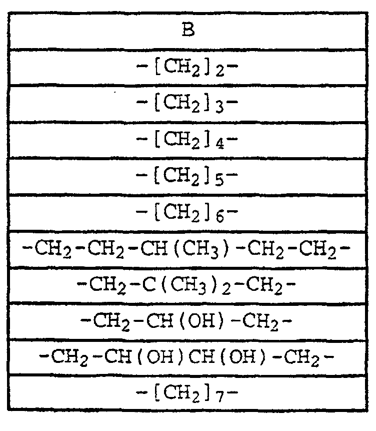

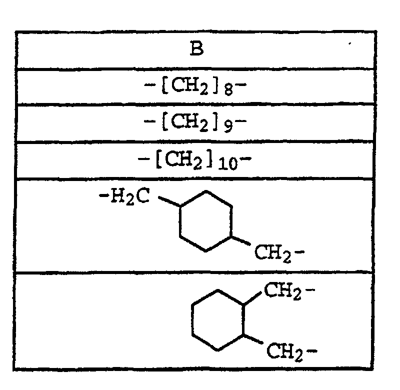

Die erfindungsgemäß zu verwendenden Verbindungen der Formel I können beispielsweise nach folgender Reaktionsgleichung 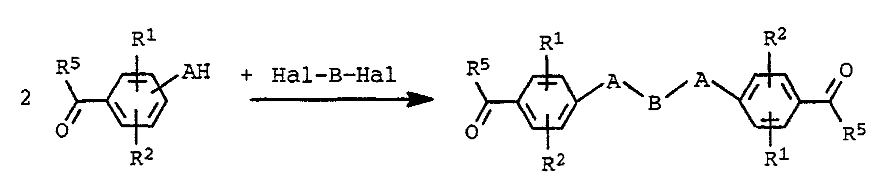 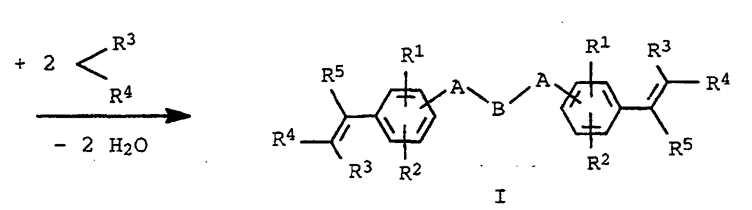 hergestellt werden, wobei Hal für Halogen wie z.B. Chlor oder Brom steht und R¹ bis R⁵ sowie die Variablen A und B die im Anspruch 1 genannte Bedeutung haben.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Bevorzugt sind solche der o.g. kosmetischen und pharmazeutischen Zubereitungen, die eine oder mehrere der Verbindungen der Formel Ia enthalten, 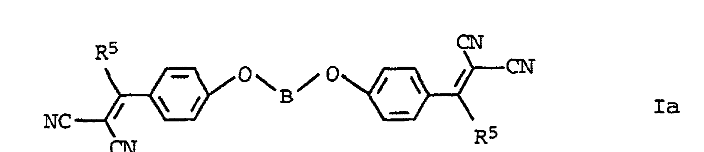 wobei die Substituenten folgende Bedeutung haben:
- R⁵: C₁-C₆-Alkyl, bevorzugt Methyl;
- B: C₁-C₁₂-Alkylen.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser -in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasserin-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen (siehe Tabelle 2):

**Tabelle 2:**

| Nr. | Stoff | CAS -Nr. (=Säure) |
|---|---|---|
| 1 | 4-Aminobenzoesäure | 150-13-0 |
| 2 | 3-(4'Trimethylammonium)-benzylidenbornan-2-on-methylsulfat | ₅₂₇₉₃₋₉₇₋₂ |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3, 3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1- (4'-Isopropylphenyl) -3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3, 3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino]carbonyl]phenyl]amino]-1,3,5-triazin-2,4-diyl]diimino]bis-,bis(2-ethylhexyl)ester | 154702-15-5 |
| 30 | 2-(2H-Benzotriazol-2-yl)-4-methyl-6-[2-methyl-3-[1,3,3,3-tetramethyl-1-[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexyl)oxy] (Aniso Triazin) | 187393-00-6 |

Schließlich sind auch mikronisierte Pigmente wie Titandioxid und Zinkoxid zu nennen.

Zum Schutz menschlicher Haare vor UV-Strahlen können die erfindungsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

### Beispiele

### I. Herstellung

### Beispiel 1

### Herstellvorschrift für die Verbindung der Nr. 2 der Tabelle 2

a) 0,5 mol 4-Hydroxyacetophenon, 0,25 mol 1,4-Dibrombutan und 0,5 mol Kaliumcarbonat wurden in 200 ml Dimethylformamid 4 h auf 80°C erhitzt. Das Gemisch wurde mit 600 ml Wasser versetzt und 30 min bei Raumtemperatur gerührt, wobei das Produkt ausfiel. Der Niederschlag wurde abgesaugt, dreimal mit je 100 ml Wasser gewaschen und bei 50°C im Vakuum (200 mbar) getrocknet. Ausbeute: 68 g (83 % d. Theorie) des dimeren Acetophenons der folgenden Formel: 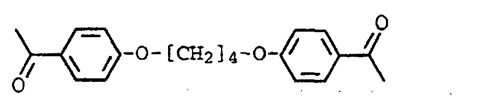
b) 65,2 g (0,2 mol) der Verbindung aus Beispiel la und 26,4 g (0,4 mol) Malonsäuredinitril wurden in 500 ml Toluol gelöst und mit je 9,2 g Phenoxyessigsäure und Salicylsäure versetzt. Man erhitzte auf 60°C und leitete bis zur Sättigung Ammoniak ein. Anschließend wurde das Reaktionsgemisch 3 h unter Rückfluß erhitzt, wobei das entstandene Reaktionswasser abgetrennt wurde. Der gebildete Niederschlag wurde abgesaugt, und mit je 100 ml Methanol und Wasser gut nachgewaschen. Anschließend wurde im Vakuum (200 mbar) bei 50°C getrocknet. Ausbeute: 73 g (86 % d. Theorie).

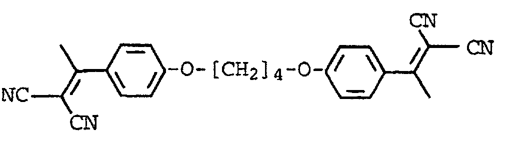

Die Herstellung der Verbindungen 1 und 3 bis 6 der Tabelle 3 erfolgt analog Beispiel 1.

### Tabelle 3: Dimere Hydroxyacetophenonkondensate

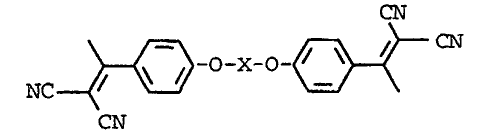

| Verbindung | X | λₘₐₓ | E¹₁ |
|---|---|---|---|
| 1 | -[CH₂]₂- | 334 | 885 |
| 2 | -[CH₂]₄- | 336 | 902 |
| 3 | -[CH₂]₆- | 336 | 780 |
| 4 | -CH₂-CH₂-CH(CH₃)-CH₂-CH₂- | 336 | 900 |
| 5 | -CH₂-C(CH₃)₂-CH₂- | 336 | 803 |
| 6 | -[CH₂]₈- | 338 | 760 |
| 7 | -[CH₂]₁₀- | 336 | 730 |

### Beispiel 2

### Standardisierte Methode zur Bestimmung der Photostabilität (Suntest)

Eine 5 gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauhten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

### Vergleich - Photostabilität:

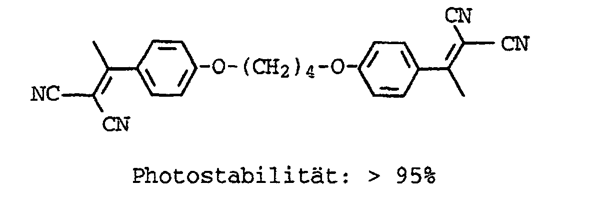

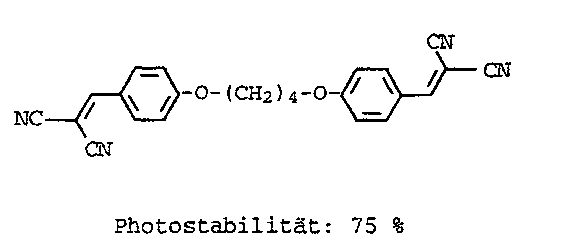

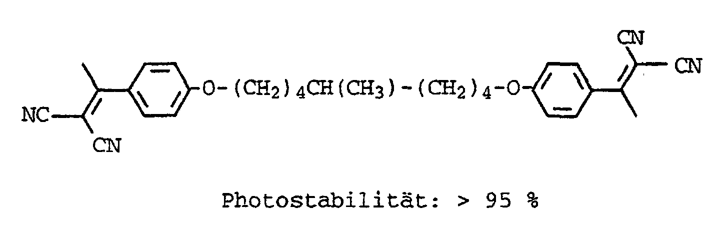

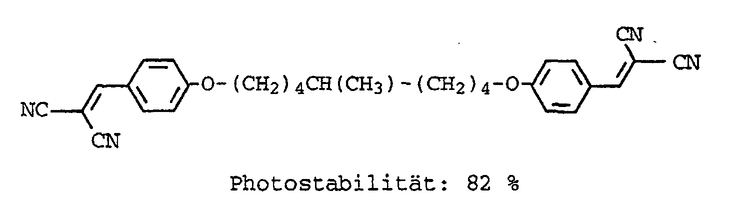

### Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 3

### Zusammensetzung für die Lippenpflege

| Massengehalt Gew.-% | |
|---|---|
| ad 100 | Eucerinum anhydricum |
| 10,00 | Glycerin |
| 10,00 | Titanium Dioxid |
| 5,00 | Verbindung Nr. 2 der Tabelle 3 |
| 8,00 | Octyl Methoxycinnamat |
| 5,00 | Zink Oxid |
| 4,00 | Castoröl |
| 4,00 | Pentaerythrityl Stearat/caprat/Caprylat/Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 4

### Zusammensetzung für die Lippenpflege

| Massengehalt Gew.-% | |
|---|---|
| ad 100 | Eucerinum anhydricum |
| 10,00 | Glycerin |
| 10,00 | Titanium Dioxid |
| 5,00 | Verbindung Nr. 4 der Tabelle 3 |
| 8,00 | Octyl Methoxycinnamat |
| 5,00 | Zink Oxid |
| 4,00 | Castoröl |
| 4,00 | Pentaerythrityl Stearat/caprat/Caprylat/Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 5

### Zusammensetzung für Sunblocker mit Mikropigmenten

| Massengehalt Gew.-% | |
|---|---|
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 2 der Tabelle 3 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 6

### Zusammensetzung für Sunblocker mit Mikropigmenten

| Massengehalt Gew.-% | |
|---|---|
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 4 der Tabelle 3 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 7

### Fettfreies Gel

| Massengehalt Gew.-% | |
|---|---|
| ad 100 | Wasser |
| 8, 00 | Octyl Methoxycinnamat |
| 7,00 | Titanium Dioxid |
| 5,00 | Verbindung Nr. 2 der Tabelle 3 |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 8

### Fettfreies Gel

| Massengehalt Gew.-% | |
|---|---|
| ad 100 | Wasser |
| 8,00 | Octyl Methoxycinnamat |
| 7,00 | Titanium Dioxid |
| 5,00 | Verbindung Nr. 4 der Tabelle 3 |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 9

### Sonnencreme (LSF 20)

| Massengehalt Gew.-% | |
|---|---|
| ad 100 | Wasser |
| 8, 00 | Octyl Methoxycinnamat |
| 8, 00 | Titanium Dioxid |
| 6,00 | PEG-7-Hydrogenated Castor Öl |
| 5,00 | Verbindung Nr. 2 der Tabelle 3 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 10

### Sonnencreme (LSF 20)

| Massengehalt Gew.-% | |
|---|---|
| ad 100 | Wasser |
| 8, 00 | Octyl Methoxycinnamat |
| 8, 00 | Titanium Dioxid |
| 6,00 | PEG-7-Hydrogenated Castor Öl |
| 5,00 | Verbindung Nr. 4 der Tabelle 3 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 11

### Sonnencreme wasserfest

| Massengehalt Gew.-% | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 2 der Tabelle 3 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 12

### Sonnencreme wasserfest

| Massengehalt Gew.-% | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 4 der Tabelle 3 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 13

### Sonnenmilch (LSF 6)

| Massengehalt Gew.-% | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 2 der Tabelle 3 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 14

### Sonnenmilch (LSF 6)

| Massengehalt Gew.-% | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 4 der Tabelle 3 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von substituierten, dimeren α-Alkyl-Styrolderivaten der Formel I, 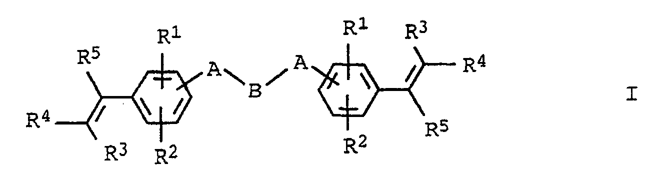 in der die Substituenten unabhängig voneinander folgende Bedeutung haben:
R¹ und R² Wasserstoff, OH, NH₂, C₁-C₁₂-Alkyl, C₁-C₁₂-Alkoxy, C₁-C₁₂-Alkylamino, C₁-C₁₂-Dialkylamino, wasserlöslich machende Substituenten, ausgewählt aus der Gruppe bestehend aus Carboxylat-, Sulfonat- oder Ammoniumresten;
R³ COOR⁶, COR⁶, CONR⁶R⁷, CN;
R⁴ COOR⁶, COR⁶, CONR⁶R⁷, CN;
R⁵ C₁-C₁₂-Alkyl,
R⁶ und R⁷ Wasserstoff, C₁-C₁₂-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
A O, S, N-R⁸;
B C₁-C₁₂-Alkylen, C₄-C₁₂-Cycloalkylen, C₈-C₂₂-Aralkylen, C₉-C₂₁-Monooxoaralkylen, [-X-]ₙ-Y;
R⁸ Wasserstoff, C₁-C₁₂-Alkyl;
X -CH₂-CH₂-Z-, -CH₂-CH₂-CH₂-Z-, -CH (CH₃)-CH₂-Z-, -CH₂-CH₂-CH₂-CH₂-Z-, -CH₂-CH (-CH₂-CH₃)-Z-;
Y -CH₂-CH₂-, -CH₂-CH₂-CH₂-, -CH(CH₃)-CH₂-, -CH₂-CH₂-CH₂-CH₂-, - CH₂-CH(CH₂-CH₃)-;
Z O, S;
n 1 bis 150,
wobei jeweils zwei der Reste R¹, R² und R⁸ zusammen mit dem aromatischen Ring, an dem sie gebunden sind, einen 5- oder 6-Ring bilden können, als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Verwendung von Verbindungen der Formel I nach Anspruch 1, wobei die Substituenten folgende Bedeutung haben:
R¹ und R² Wasserstoff, C₁-C₈-Alkyl, C₁-C₈-Alkoxy, wasserlöslich machende Substituenten, ausgewählt aus der Gruppe bestehend aus Carboxylat-, Sulfonat- oder Ammoniumresten;
R³ COOR⁶, CN;
R⁴ COOR⁶, CN;
R⁵ C₁-C₆-Alkyl;
R⁶ C₁-C₁₂-Alkyl;
A O;
B C₁-C₁₂-Alkylen, C₈-C₂₂-Aralkylen, C₄-C₁₂-Cycloalkylen, [-X-]ₙ-Y;
X -CH₂-CH₂-O-, -CH₂-CH₂-CH₂-O-, -CH(CH₃)-CH₂-O-, -CH₂-CH₂-CH₂-CH₂-O-, -CH₂-CH₂-CH₂-CH₂-O-,
Y -CH₂-CH₂-, -CH₂-CH₂-CH₂-, -CH(CH₃)-CH₂-, -CH₂-CH₂-CH₂-CH₂-, -CH₂-CH(CH₂-CH₃)-;
n 1 bis 20.

3. Verwendung von Verbindungen der Formel I nach einem der Ansprüche 1 oder 2, wobei die Substituenten folgende Bedeutung haben:
R¹ und R² Wasserstoff, C₄-C₈-Alkyl, C₄-C₈-Alkoxy, wasserlöslich machende Substituenten, ausgewählt aus der Gruppe bestehend aus Carboxylat-, Sulfonat- oder Ammoniumresten;
R³ und R⁴ CN;
R⁵ C₁-C₆-Alkyl;
A O;
B C₁-C₁₂-Alkylen, [-X-]ₙ-Y;
X -CH₂-CH₂-O-, -CH(CH₃)-CH₂-O-;
Y CH₂-CH₂-O-, -CH(CH₃)-CH₂-;
n 1 bis 20.

4. Verwendung von substituierten, dimeren α-Alkyl-Styrolderivaten nach einem der Ansprüche 1 bis 3 mit der folgenden Strukturformel Ia, 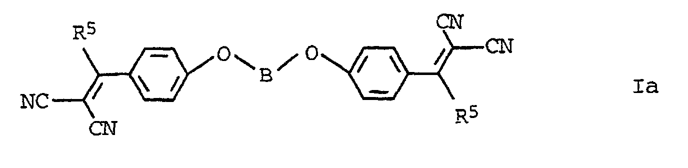 wobei die Substituenten folgende Bedeutung haben:
R⁵ C₁-C₆-Alkyl;
B C₁-C₁₂-Alkylen.

5. Verwendung von Verbindungen der Formel I nach einem der Ansprüche 1 bis 4 als photostabile UV-A-Filter.

6. Verwendung von Verbindungen der Formel I, definiert gemäß Anspruch 1, als UV-Stabilisator in kosmetischen und pharmazeutischen Formulierungen.

7. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit für kosmetische und pharmazeutische Zubereitungen bekannten im UV-Bereich absorbierenden Verbindungen, als photostabile UV-Filter wirksame Mengen von Verbindungen der Formel I 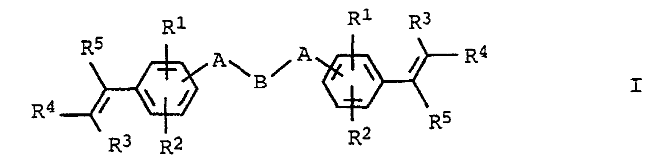 enthalten, in der die Substituenten die Bedeutung gemäß Anspruch 1 haben.

8. Lichtschutzmittel enthaltende kosmetische und pharmazeutische Zubereitungen nach Anspruch 7, enthaltend als UV-A-Filter Verbindungen der Formel Ia, in der die Substituenten die Bedeutung definiert gemäß Anspruch 4 haben.

## Claims

1. The use of substituted, dimeric α alkylstyrene derivatives of the formula I 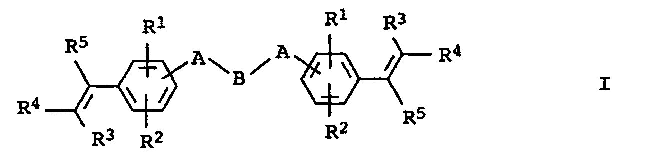 in which the substituents independently of one another have the following meanings:
R¹ and R² are hydrogen, OH, NH₂, C₁-C₁₂-alkyl, C₁-C₁₂-alkoxy, C₁-C₁₂-alkylamino, C₁-C₁₂-dialkylamino, substituents which confer solubility in water, chosen from the group consisting of carboxylate, sulfonate or ammonium radicals;
R³ is COOR⁶, COR⁶, CONR⁶R⁷, CN;
R⁴ is COOR⁶, COR⁶, CONR⁶R⁷, CN;
R⁵ is C₁-C₁₂-alkyl,
R⁶ and R⁷ are hydrogen, C₁-C₁₂-alkyl, C₂-C₁₀-alkenyl, C₃-C₁₀-cycloalkyl, C₃-C₁₀-cycloalkenyl, aryl, heteroaryl, optionally substituted;
A is O, S, N-R⁸;
B is C₁-C₁₂-alkylene, C₄-C₁₂-cycloalkylene, C₈-C₂₂-aralkylene, C₉-C₂₁-monooxoaralkylene, [-X-]ₙ-Y;
R⁸ is hydrogen, C₁-C₁₂-alkyl;
X is -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
Y is -CH₂-CH₂-, -CH₂-CH₂-CH₂-, -CH(CH₃)-CH₂-, -CH₂-CH₂-CH₂-CH₂-, -CH₂-CH(CH₂-CH₃)-;
Z is O, S;
n is 1 to 150,
where, in each case, two of the radicals R¹, R² and R⁸ together with the aromatic ring to which they are bonded can form a 5- or 6-membered ring, as photostable UV filters in cosmetic and pharmaceutical preparations for protecting human skin or human hair from solar rays, alone or together with compounds which absorb in the UV region and which are known per se for cosmetic and pharmaceutical preparations.

2. The use of the compounds of the formula I as claimed in claim 1, where the substituents have the following meanings:
R¹ and R² are hydrogen, C₁-C₈-alkyl, C₁-C₈-alkoxy, substituents which confer solubility in water, chosen from the group of carboxylate, sulfonate or ammonium radicals;
R³ is COOR⁶, CN;
R⁴ is COOR⁶, CN;
R⁵ is C₁-C₆-Alkyl;
R⁶ is C₁-C₁₂-Alkyl;
A is 0;
B is C₁-C₁₂-alkylene, C₈-C₂₂-aralkylene, C₄-C₁₂-cycloalkylene, [-x-]ₙ-Y;
X is -CH₂-CH₂-O-, -CH₂-CH₂-CH₂-O-, -CH(CH₃)-CH₂-O-, - CH₂-CH₂-CH₂-CH₂-O-, -CH₂-CH(CH₂-CH₃)-0-;
Y is -CH₂-CH₂-, -CH₂-CH₂-CH₂-, -CH(CH₃)-CH₂-, - CH₂-CH₂-CH₂-CH₂-, -CH₂-CH(CH₂-CH₃)-;
n is 1 to 20.

3. The use of compounds of the formula I as claimed in either of claims 1 or 2, where the substituents have the following meanings:
R¹ and R² are hydrogen, C₄-C₈-alkyl, C₄-C₈-alkoxy, substituents which confer solubility in water, chosen from the group consisting of carboxylate, sulfonate or ammonium radicals;
R³ and R⁴ are CN;
R⁵ is C₁-C₆ alkyl;
A is O;
B is C₁-C₁₂-alkylene, [-X-]ₙ-Y;
X is -CH₂-CH₂-O-, -CH(CH₃)-CH₂-O-;
Y is -CH₂-CH₂-, -CH(CH₃)-CH₂-;
n is 1 to 20.

4. The use of substituted, dimeric α alkylstyrene derivatives as claimed in any of claims 1 to 3 having the following structural formula Ia 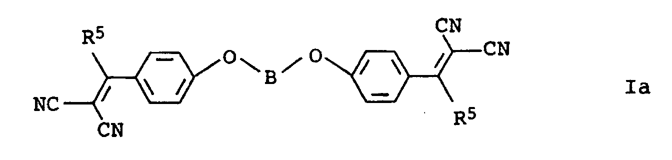 where the substituents have the following meanings:
R⁵ is C₁-C₆-alkyl;
B is C₁-C₁₂-alkylene.

5. The use of compounds of the formula I as claimed in any of claims 1 to 4 as photostable UV-A filters.

6. The use of compounds of the formula I, defined as in claim 1, as UV stabilizer in cosmetic and pharmaceutical formulations.

7. A cosmetic or pharmaceutical preparation comprising suncreens for protecting the human epidermis or human hair from UV light in the range from 280 to 400 nm, which comprises, in a cosmetically or pharmaceutically suitable carrier, alone or together with compounds which absorb in the UV region and are known for cosmetic and pharmaceutical preparations, an effective amount of compounds of the formula I 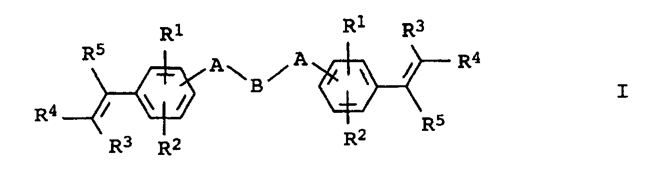 in which the substituents are as defined in claim 1, as photostable UV filters.

8. A cosmetic or pharmaceutical preparation comprising sunscreens as claimed in claim 7, comprising, as UV-A filter, compounds of the formula Ia in which the substituents are as defined in claim 4.

## Revendications

1. Utilisation d'alpha-alkyl-styrènes dimères substitués répondant à la formule I 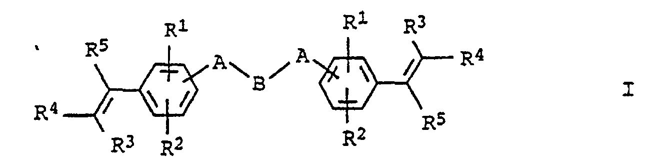 dans laquelle les symboles ont, indépendamment les uns des autres, les significations suivantes :
R¹ et R² : l'hydrogène, des groupes OH, NH2, alkyle en C1-C12, alcoxy en C1- C12, alkylamino en C1-C12, di-(alkyle en C1-C12)amino, des substituants hydrosolubilisants choisis parmi les groupes carboxylate, sulfonate et ammonium ;
R³ : COOR⁶, COR⁶, CONR⁶R⁷, CN ;
R⁴ : COOR⁶, COR⁶, CONR⁶R⁷, CN ;
R⁵ : un groupe alkyle en C1-C12,
R⁶ et R⁷ : l'hydrogène, des groupes alkyle en C1-C12, alcényle en C2-C10, cycloalkyle en C3-C10, cycloalcényle en C3-C10, aryle, hétéroaryle, éventuellement substitués ;
A : O, S, N-R⁸ ;
B : un groupe alkylène en C1-C12, cycloalkylène en C4-C12, aralkylène en C8-C22, monooxoaralkylène en C9-C21, [-X-]ₙ-Y;
R⁸ : l'hydrogène, un groupe alkyle en C1-C12 ;
X: -CH₂-CH₂-Z-, -CH₂-CH₂-CH₂-Z-, -CH(CH₃)-CH₂-Z, -CH₂-CH₂- CH₂-CH₂-Z-, -CH₂-CH(CH₂-CH₃)-Z- ;
Y : -CH₂-CH₂-, -CH₂-CH₂-CH₂-, -CH(CH₃)-CH₂-, -CH₂-CH₂-CH₂- CH₂-, -CH₂-CH(CH₂-CH₃)- ;
Z : O, S ;
n : 1 à 150.
deux des groupes R¹, R² et R³ pouvant former avec le cycle aromatique auquel ils sont reliés un cycle à cinq ou six chaînons, en tant que filtres UV photostables dans des compositions cosmétiques et pharmaceutiques pour la protection de la peau humaine ou de la chevelure humaine contre les rayons solaires, seuls ou avec des composés connus en soi pour les compositions cosmétiques et pharmaceutiques et absorbant dans le domaine UV.

2. Utilisation de composés de formule I selon la revendication 1, dans laquelle les symboles ont les significations suivantes :
R¹ et R² : l'hydrogène, des groupes alkyle en C1-C8, alcoxy en C1-C8, des substituants hydrosolubilisants choisis parmi les groupes carboxylate, sulfonate et ammonium ;
R³ : COOR⁶, CN ;
R⁴ : COOR⁶, CN ;
R⁵ : alkyle en C1-C6 ;
R⁶ : alkyle en C1-C12;
A : O ;
B : alkylène en C1-C12, aralkylène en C8-C22, cycloalkylène en C4- C12, [-X-]ₙ-Y;
X : -CH₂-CH₂-O-, -CH₂-CH₂-CH₂-O-, -CH(CH₃)-CH₂-O-, -CH₂-CH₂- CH₂-CH₂-O-, -CH₂-CH(CH₂-CH₃)-O-;
Y : -CH₂-CH₂-, -CH₂-CH₂-CH₂-, -CH(CH₃)-CH₂-, -CH₂-CH₂-CH₂- CH₂-, -CH₂-CH(CH₂-CH₃)- ;
n: 1 à 20.

3. Utilisation de composés de formule I selon l'une des revendications 1 ou 2, pour lesquels les symboles ont les significations suivantes :
R¹ et R² : l'hydrogène, des groupes alkyle en C4-C8, alcoxy en C4-C8, des substituants hydrosolubilisants choisis parmi les groupes carboxylate, sulfonate et ammonium;
R³ et R⁴ : CN;
R⁵ : alkyle en C1-C6 ;
A : O;
B : alkylène en C1-C12, [-X-]ₙ-Y ;
X : -CH₂-CH₂-O-, -CH(CH₃)-CH₂-O- ;
Y : -CH₂-CH₂-,-CH(CH₃)-CH₂-;
n: 1 à 20.

4. Utilisation d'alpha-alkyl-styrènes dimères substitués selon l'une des revendications 1 à 3, répondant à la formule de structure la ci-après : 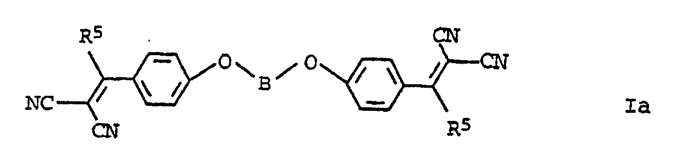 dans laquelle les symboles ont les significations suivantes :
R⁵ : alkyle en C1-C6 ;
B : alkylène en C1-C12.

5. Utilisation de composés de formule I selon l'une des revendications 1 à 4 en tant que filtres photostables pour UV-A.

6. Utilisation de composés de formule I tels que définis dans la revendication 1, en tant que stabilisants contre les UV dans des compositions cosmétiques et pharmaceutiques.

7. Compositions cosmétiques et pharmaceutiques contenant des agents de protection contre la lumière et prévues pour la protection de l'épiderme humain ou de la chevelure humaine contre la lumière UV du domaine de 280 à 400 nm, **caractérisées par le fait qu'**elles contiennent, dans un véhicule approprié pour les usages cosmétiques et pharmaceutiques, seuls ou avec des composés connus pour les compositions cosmétiques et pharmaceutiques et absorbant dans le domaine UV, des quantités efficaces en tant que filtres UV photostables, de composés de formule I 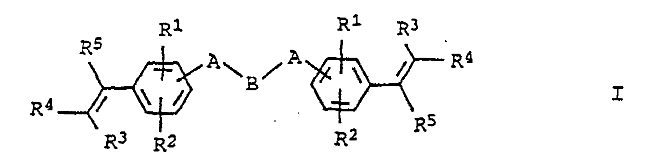 dans laquelle les symboles ont les significations indiquées dans la revendication 1.

8. Compositions cosmétiques et pharmaceutiques contenant des agents de protection contre la lumière selon la revendication 7, qui contiennent en tant que filtres contre l'UV-A des composés de formule Ia dans laquelle les symboles ont les significations indiquées dans la revendication 4.
